**Europäisches Patentamt**

**European Patent Office**

**Office européen des brevets**

(19)

(11) Veröffentlichungsnummer: **0 122 374**
**B1**

(12) # EUROPÄISCHE PATENTSCHRIFT

(45) Veröffentlichungstag der Patentschrift:
**23.09.87**

(21) Anmeldenummer: **84101171.1**

(22) Anmeldetag: **06.02.84**

(51) Int. Cl.⁴: **C 07 C 37/60,** C 07 C 39/08,
C 07 C 39/15, C 07 C 41/26,
C 07 C 43/20

(54) Verfahren zur Herstellung von Dihydroxybenzolen.

(30) Priorität: **11.03.83 DE 3308769**

(43) Veröffentlichungstag der Anmeldung:
**24.10.84 Patentblatt 84/43**

(45) Bekanntmachung des Hinweises auf die Patenterteilung:
**23.09.87 Patentblatt 87/39**

(84) Benannte Vertragsstaaten:
**AT BE CH DE FR GB IT LI LU NL SE**

(56) Entgegenhaltungen:
**DE-A-2 348 957**
**DE-A-2 410 758**

(73) Patentinhaber: **Degussa Aktiengesellschaft,
Weissfrauenstrasse 9, D-6000 Frankfurt am Main
1 (DE)**

(72) Erfinder: **Drauz, Karlheinz, Dr. Dipl.- Chem.,
Flurstrasse 5, D-6463 Freigericht 1 (DE)**
Erfinder: **Kleemann, Axel, Dr. Dipl.- Chem.,
Greifenhagenstrasse, D-6450 Hanau 9 (DE)**

EP 0 122 374 B1

LIBER, STOCKHOLM 1987

## Beschreibung

Die Erfindung betrifft die Herstellung von Dihydroxybenzolen sowie deren Monoäthern durch Kernhydroxylierung der entsprechenden Phenole oder Phenoläther mit Wasserstoffperoxid.

Wichtige Dihydroxybenzole sind Abkömmlinge von Phenol, den Naphtholen, aber auch von Anthracen oder Phenanthren. Sie lassen sich bei der Herstellung von Farbstoffen, in der kunststoffproduktion, bei der Photographie oder zur Herstellung wichtiger Pflanzenschutzmittel einsetzen.

Ihre Herstellung war daher schon lange Gegenstand eingehender Untersuchungen. Die Hydroxylierung wurde sowohl mit Wasserstoffperoxid selbst, wie auch mit Hydroperoxiden, Peroxiden oder auch Persäuren, wie z. B. Perameisen- oder Peressigsäure, durchgeführt. Doch war Wasserstoffperoxid, da es am leichtesten zugänglich war, bevorzugt, da mit Percarbonsäuren, Hydroperoxiden, Peroxiden Nebenreaktionen auftraten, (EP-A-0 027 593).

Stets war bei diesen Hydroxylierungen ein Katalysator anwesend. Dieser Katalysator konnte ein Metalloid, wie Schwefel, Selen, Tellur, Phosphor, Arsen oder Antimon in elementarer Form sein (DE-OS 23 48 957), oder man verwendete Borverbindungen (DE-PS 1 543 830).

Verschiedene Verfahren arbeiteten mit Übergangselementen in Form ihrer Ionen (DE-OS 21 62 552), besonders mit Eisenionen (DE-OS 21 62 589 oder DE-PS 24 07 398) oder Kobaltionen (DE-AS 23 41 743), oder auch mit den entsprechenden Oxiden (US-PS 2 395 638).

Ausserdem wurden starke Säuren, wie Schwefelsäure, Sulfonsäuren (DE-OS 21 38 735, DE-AS 24 10 742, DE-AS 24 10 758, DE-AS 24 62 967) oder Gemische aus Schwefel- und Phosphorsäure (DE-OS 21 38 735) eingesetzt, bzw. wurden in dieser letztgenannten Offenlegungsschrift organische Säuren, wie u.a. Trichloressigsäure oder Weinsäure genannt.

Die schon genannten Percarbonsäuren dienten ebenfalls als Katalysatoren (FR-PS 1 479 354). Bei den Katalysatoren handelte es sich in allen genannten Fällen um feste oder flüssige Substanzen. Wasserstoffperoxid - als bevorzugtes Oxydationsmittel - wurde meist in wässriger Lösung verschiedener Konzentrationen bis hin zu sehr hohen, explosionsgefährlichen Konzentrationen eingesetzt; so arbeitete das Verfahren nach der DE-PS 20 64 497 mit Lösungen, die nur noch 5 Gew.-% Wasser enthielten, aber selbst bei diesem höchstkonzentriertem Wasserstoffperoxid lag die Ausbeute an Dihydroxyderivaten nur bei 70 % und sank entsprechend der Verdünnung des Wasserstoffperoxids erheblich ab.

Hinzu kam, dass in diesem wie auch in anderen Verfahren mit einem sehr grossen Überschuss an dem zu hydroxylierenden Phenol gearbeitet werden musste, um überhaupt die oben angegebene Ausbeute zu erhalten. Wurde dieser Überschuss gesenkt, z. B. von 20 Mol auf 10 Mol pro Mol Wasserstoffperoxid, so sank trotz hoher Konzentration an Wasserstoffperoxid die Ausbeute drastisch.

Bekanntlich erfordern aber derartige Überschüsse an einer Reaktionskomponenten, die ja zurückgeführt werden muss, zusätzliche technische Aufwendungen; vor allem hinsichtlich der Grösse der einzusetzenden Apparaturen.

Da man immer bemüht ist, grosse Überschüsse an einer Komponente nach Möglichkeit zu vermeiden, wurde versucht, den Einsatz wäßriger Lösungen von Wasserstoffperoxid zu umgehen.

So wurden schon verschiedentlich Lösungen von Wasserstoffperoxid in organischen Lösungsmitteln verwendet. Man arbeitete z. B. nach dem Verfahren der DE-PS 24 10 758 bevorzugt mit Wasserstoffperoxidlösungen in Abkömmlingen der Phosphor- oder Phosphonsäure, und zwar in Gegenwart einer starken Säure, wie Schwefelsäure (100 %ig) oder Fluorsulfonsäure.

Diese hochkonzentrierten starken Säuren haben aber den Nachteil, dass ihre Abtrennung aus dem Reaktionsgemisch Schwierigkeiten bereitet (DE-AS 26 58 943), vor allem, da ihre Konzentration im Reaktionsgemisch die Reaktionsdauer erheblich beeinflusst.

Die Überschüsse an Phenolen waren zwar gegenüber denen im Verfahren der DE-AS 20 64 497 etwas vermindert, aber dies wog den Nachteil durch die starken Säuren nicht auf.

Eine zusätzliche Schwierigkeit beim Verfahren der DE-PS 24 10 758 bei der Aufarbeitung des Reaktionsgemisches wurde durch die Anwesenheit des nach der Reaktion mit Wasserstoffperoxid gebildeten Wassers hervorgerufen.

Da die eingesetzten Lösungsmittel für Wasserstoffperoxid zum Teil höher siedeten als die eingesetzten Phenole und diese oft - vor allem auch Phenol selbst - mit Wasser Azeotrope bildeten, deren Siedepunkte unter denen der organischen Lösungsmittel lagen, war eine einwandfreie Abtrennung der überschüssigen Phenole aus dem Reaktionsgemisch äusserst problematisch.

Man ging daher andere Wege und versuchte, zunächst einmal ohne Katalysator, d.h. vor allem ohne die starken Säuren, auszukommen. Da die Katalysatoren in erster Linie für die Aktivierung von Wasserstoffperoxid notwendig waren, wurde im Verfahren der DE-AS 26 58 843 mit organischen Lösungen von percarbonsäuren gearbeitet. Ein zusätzlicher Katalysator wurde nicht verwendet.

Ganz abgesehen davon, dass das genannte Verfahren eine vollständige Anlage zur Herstellung einer organischen Percarbonsäure, die zunächst aus Wasserstoffperoxid und Carbonsäure gewonnen und darauf durch Extraktion dieser sog. "Gleichgewichtssäure" aus ihrem wäßrigen Medium hergestellt wird, voraussetzt, hatte sich gezeigt, dass eine angeblich gute Selektivität und gute Ausbeute nur durch Anwesenheit zusätzlicher Persäurestabilisatoren möglich war (DE-OS 23 64 181; EP-A-0 027 593).

Auch der Versuch, Brenzcatechin und Hydrochinon ohne Katalysator mit dampfförmigem Wasserstoffperoxid herzustellen, war wegen der Explosionsgefährlichkeit schlecht technisch durchführbar (JP-OS 24056/1974).

Nach dem Obengesagten ergibt sich, dass Verfahren, die Wasserstoffperoxid als das einfachste und am besten zugängliche Hydroxylierungsmittel verwendeten, bei der technischen Herstellung von Dihydroxybenzolen keine insgesamt befriedigende Verfahrensweise ermöglichten.

Daher wurden in neuerer Zeit nur Verfahren entwickelt, die nicht direkt Wasserstoffperoxid verwendeten und aus dem Grund teilweise hohe technische Aufwendungen notwendig machten.

Ziel der Erfindung ist es daher, die Kernhydroxylierung von substituierten Phenolen oder deren Äthern mit Wasserstoffperoxid in Gegenwart von Katalysatoren in technisch einfacher Weise und mit sehr guten Ausbeuten durchzuführen.

Es wurde nun gefunden, daß sich diese Aufgabe lösen läßt bei Verwendung von wasserfreien organischen Lösungen von Wasserstoffperoxid, wenn man die Umsetzung bei Temperaturen von 20 - 200°C mit Lösungen von Wasserstoffperoxid, die wasserfrei sind, d.h. 0 - 1 Gew.-%, bevorzugt unter 0,5 Gew.-%, Wasser enthalten, die mit organischen Lösungsmitteln hergestellt werden, die mit Wasser Azeotrope bilden, deren Azeotropsiedepunkte unter dem Siedepunkt von Wasserstoffperoxid, bezogen auf Normaldruck, liegen, sowie in Gegenwart von Schwefeldioxid in Mengen von 0,0001 bis 0,1 Mol, bezogen auf 1 Mol Wasserstoffperoxid, durchführt, wobei das Molverhältnis von Phenol bzw. Phenoläther zu Wasserstoffperoxid bei 5 bis 20 : 1 liegt.

Als Lösungsmittel kommen auch z. B. in Frage Äther, wie Dioxan, Diisopropyläther, Methyl-tert.-butyläther.

Bevorzugte Lösungsmittel sind Alkyl- oder Cycloalkylester von gesättigten, aliphatischen Carbonsäuren, die eine Gesamtkohlenstoffzahl von 4 - 8 besitzen.

Besonders geeignete Ester sind die der Essig- oder Propionsäure, vor allem Essigsäure-n- oder -i-propylester. Es können auch Gemische der Ester eingesetzt werden.

Die organischen Wasserstoffperoxidlösungen können in üblicher Form stabilisiert sein, siehe ULLMANN, Enzyklopädie der technischen Chemie, 4. Aufl., Band 17, Seite 709.

Die genannten Lösungen von Wasserstoffperoxid in Alkyl- oder Cycloalkylestern werden nach dem Verfahren der DE-A-32 25 307.9 gewonnen.

Das als Katalysator wirkende Schwefeldioxid kann in gasförmigem Zustand verwendet werden. Schwefeldioxid kann aber auch in einem beliebigen Lösungsmittel gelöst sein, das sowohl mit Schwefeldioxid selbst wie auch mit Wasserstoffperoxid keine störenden Reaktionen eingeht, beispielsweise in Dialkyläthern, Estern der Phosphor- oder Phosphonsäure. Die Konzentrationen richten sich nach der Löslichkeit von SO₂ im Lösungsmittel, im allgemeinen liegen sie bei 0,1 bis 50, bevorzugt bei 1 bis 10 Gew.-%. Günstig ist es aber, Schwefeldioxid als Lösung in einem der oben beschriebenen Carbonsäureester einzusetzen. Schwefeldioxid wird in sehr geringen Mengen verwendet, bevorzugt von 0,0005 bis 0,01 Mol, bezogen auf 1 Mol Wasserstoffperoxid, vor allem verglichen mit durch Protonensäuren sauer katalysierten Hydroxylierungen.

Die Umsetzung findet bevorzugt bei Temperaturen von 40 bis 180°C statt.

Die organischen Lösungen von Wasserstoffperoxid in den genannten Alkyl- oder Cycloalkylestern ermöglichen höhere Konzentrationen (bis über 60 Gew.-%).

Das erfindungsgemässe Verfahren lässt sich - wie gesagt für die Kernhydroxylierung von substituierten Phenolen sowie deren Monoäthern durchführen. So lassen sich z. B. Alkylabkömmlinge von Phenol hydroxylieren, z. B. Kresole, Äthyl- oder Butylphenole, wie auch Alkoxyverbindungen, wie Anisol, ferner deren Alkyl- oder Halogenabkömmlinge, ebenso Arylphenole, wie 4-Hydroxybiphenyl.

Selbstverständlich sind auch die Halogenverbindungen des Phenols selbst oder auch Alkoxyverbindungen des Phenols selbst einsetzbar.

Von den Phenyläthern seien besonders Phenyläthyläther, Phenylisopropyläther oder p-Kresolmethyläther noch erwähnt.

Der Druck ist für die Umsetzung nicht entscheidend; sie wird im allgemeinen bei Normaldruck ausgeführt.

Die Reaktionsdauer hängt ab von der Temperatur und der Konzentration an Schwefeldioxid.

Um die beste Reaktionszeit festzustellen, kann man einen Handversuch durchführen.

Vorzugsweise wird so verfahren, dass nach 30 Minuten bereits mehr als 95 % des eingesetzten Wasserstoffperoxids umgesetzt sind.

Die Hydroxylierung der substituierten Phenole oder Phenoläther nach dem erfindungsgemässen Verfahren gelingt dann besonders gut, wenn wasserfreie Lösungen von Wasserstoffperoxid in den genannten Carbonsäureestern mit einem Gewichtsverhältnis von etwa 1 : 4 bis 2 : 1 H₂O₂/Carbonsäureester eingesetzt werden.

Dieses Gewichtsverhältnis erreicht man auch bei Verwendung von niedriger konzentrierten H₂O₂-Lösungen, indem man aus diesen Lösungen im Gemisch mit dem Phenolderivat den Carbonsäureester über Kopf abdestilliert. Die Entnahme kann auf jeden beliebigen Wert einreguliert werden.

Bei der Destillation wird so verfahren, dass praktisch kein Phenol und kein Wasserstoffperoxid mit ausgetragen werden.

Als positiver Nebeneffekt findet noch eine azeotrope Entwässerung des Phenols statt, da das Azeotrop Wasser/Carbonsäureester die niedrigst siedende Komponente ist.

Die Aufbereitung des Reaktionsgemisches ist wesentlich einfacher als es bisher bekannt war.

Da die erfindungsgemäß als Lösungsmittel zu verwendenden Ester niedriger als die zur Umsetzung kommenden Phenole sieden, wird als erstes ein Azeotrop zwischen dem Ester und Wasser abdestilliert. Die Schwierigkeiten einer Wasser-Phenoltrennung, wie sie bisher aufgetreten sind, fallen fort. Dies ist besonders wichtig, da die Phenole im Überschuß angewendet werden und wieder zurückgeführt werden mussen.

Bei der Aufarbeitung ist es nicht unbedingt notwendig, infolge der extrem niedrigen Katalysatorkonzentrationen, vor einer destillativen Trennug eine Abtrennung des Katalysators, beispielsweise durch Neutralisation, vorzunehmen; das rohe Reaktionsgemisch wird direkt einer Destillation unterworfen.

Das Molverhältnis von substituiertem Phenol bzw. Phenoläther zu Wasserstoffperoxid liegt bevorzugt bei 5 bis 15 : 1, besonders günstig bei 10 : 1.

Beim erfindungsgemäßen Verfahren wird zwar ein Katalysator eingesetzt, aber in solch geringen Mengen, daß seine gesonderte Abtrennung vor der destillativen Aufarbeitung des Reaktionsgemisches überflüssig wird, wie oben gesagt.

Hinzu kommt nun, daß eine sehr günstige Raum-Zeit-Ausbeute aufgrund der kurzen Reaktionszeiten erzielt wird; für die technische Durchführung genügen also kleine Reaktionsvolumina. So liegen 99 %ige Umsätze und mehr schon nach 20 bis 30 Minuten vor. Durch die kurze Reaktionsdauer ist außerdem gleichzeitig die mögliche Gefahr von Zersetzungen vermindert. Diese Reaktion läßt sich auch gut kontinuierlich durchführen.

Wesentlich ist ferner, daß die leichtsiedenden Lösungsmittel gemeinsam mit dem vorhandenen Wasser einwandfrei von den restlichen Phenolen und den Reaktionsprodukten abgetrennt werden können, so dass das Phenol praktisch wasserfrei wieder in die Umsetzungsstufe zurückgeführt werden kann.

Alle diese Vorteile sind nicht mit einer Verringerung der bisher nach dem Stand der Technik erhaltenen Ausbeuten verbunden, sondern die Ausbeuten selbst sind erhöht.

Als besonders günstig erwiesen sich frisch hergestellte Lösungen von Schwefeldioxid.

Die Erfindung wird anhand nachstehender Beispiele näher erläutert.

**Beispiel 1**

150,2 g (1,0 mol) 4-tert.Butylphenol werden auf 101°C erwärmt.

Zu der gerührten Schmelze setzt man 0,4 g einer 4,8 gew.-%igen Lösung von Schwefeldioxid in Essigsäure-n-propylester zu und anschliessend 6,37 g einer 53,4 gew.-%igen wasserfreien Lösung von Wasserstoffperoxid in Essigsäure-n-propylester (0,1 mol).

Die Temperatur in der Reaktionslösung erhöht sich danach auf 155°C.

Nach Abklingen der Exotherme wird nach 20 min ein Wasserstoffperoxidumsatz von 99,4 % bestimmt. Das Reaktionsgemisch enthält dann 13,4 g 4-tert. Butylbrenzkatechin, was einer Ausbeute von 81,1 %, bezogen auf das umgesetzte Wasserstoffperoxid, entspricht.

**Beispiel 2**

108,1 g (1,0 mol) p-Kresol werden auf 90°C erwärmt.

Zu der gerührten Schmelze setzt man 0,4 g einer 4,8 g gew.-%igen Lösung von Schwefeldioxid in Essigsäuren-propylester zu und anschliessend 11,15 g einer 30,5 gew.-%igen wasserfreien Lösung von Wasserstoffperoxid in Essigsäure-n-propylester (0,1 mol).

Die Temperatur in der Reaktionslösung erhöht sich danach auf 149°C

Nach Abklingen der Exotherme wird nach 15 min ein Wasserstoffperoxidumsatz von 99,8 % bestimmt. Das Reaktionsgemisch enthält dann 8,84 g (71,2 mMol) 4-Methylbrenzkatechin und 1,11 g (8,9 mMol) 4-Methylresorcin, was einer Ausbeute an Dihydroxybenzolen von 80,3 %, bezogen auf das umgesetzte Wasserstoffperoxid, entspricht.

**Beispiel 3**

170,2 g (1,0 mol) 4-Hydroxybiphenyl in 300 ml Essigsäure-n-propylester werden auf 80°C erwärmt.

Zu der stark gerührten Reaktionsmischung setzt man 0,6 g einer 4,8 gew.-%igen Lösung von Schwefeldioxid in Essigsäure-n-propylester zu und anschliessend 6,37 g einer 53,4 gew.-%igen wasserfreien Lösung von Wasserstoffperoxid in Essigsäure-n-propylester- (0,1 Mol $H_2O_2$).

Die Temperatur in der Reaktionslösung erhöht sich danach auf 128°C.

Nach Abklingen der Exotherme wird nach 30 min ein Wasserstoffperoxidumsatz von 98,7 % bestimmt. Das Reaktionsgemisch enthält dann 10,2 g (59,9 mMol) 1,2-Dihydroxy-4-phenylbenzol, was einer Ausbeute von 60,7 %, bezogen auf das umgesetzte Wasserstoffperoxid, entspricht.

**Beispiel 4**

108,1 g (1,0 mol) Anisol werden auf 80°C erwärmt.

Zu der gerührten Lösung setzt man 0,4 g einer 4,8 gew.-%igen Lösung von Schwefeldioxid in Essigsäure-n-propylester zu und anschliessend 6,37 g einer 53,4 gew.-%igen wasserfreien Lösung von Wasserstoffperoxid in Essigsäure-n-propylester (0,1 mol).

Die Temperatur in der Reaktionsmischung steigt auf 140°C. Nach 30 min wird ein $H_2O_2$-Umsatz von 97,9 % bestimmt. Das Reaktionsgemisch enthält dann 5,28 g (42,5 mMol) Brenzkatechinmonomethyläther und 2,36 g (19,0 mMol) Hydrochinonmonomethyläther, was einer Ausbeute von 63,1 %, bezogen auf das umgesetzte Wasserstoffperoxid, entspricht.

**Beispiel 5**

122,2 g (1,0 mol) 4-Äthylphenol werden auf 70°C erwärmt.

Zu der gerürten Schmelze setzt man 0,4 g einer 4,8 gew.-%igen Lösung von Schwefeldioxid in Essigsäure-n-propylester zu und anschliessend 11,15 g einer 30,5 gew.-%igen wasserfreien Lösung von Wasserstoffperoxid in Essigsäure-n-propylester (0,1 mol).

Die Temperatur in der Reaktionslösung erhöht sich danach auf 150°C.

Nach Abklingen der Exotherme wird nach 15 min ein Wasserstoffperoxidumsatz von 99,5 % bestimmt.

Das Reaktionsgemisch enthält dann 7,24 g (52,4 mMol) 4-Äthylbrenzkatechin und 1,79 g (12,95 mMol) 4-Äthylresorcin, was einer Ausbeute an Dihydroxybenzolen von 65,6 %, bezogen auf das eingesetzte $H_2O_2$, entspricht.

**Beispiel 6**

108,1 g (1,0 mol) o-Kresol werden auf 90°C erwärmt.

Zu der gerührten Schmelze setzt man 0,4 g einer 4,8 gew.-%igen Lösung von Schwefeldioxid in Essigsäure-n-propylester zu und anschliessend 11,15 g einer 30,5 gew.-%igen wasserfreien Lösung von Wasserstoffperoxid in Essigsäure-n-propylester (0,1 mol).

Die Temperatur in der Reaktionslösung erhöht sich danach auf 146°C

Nach Abklingen der Exotherme wird nach 20 min ein Wasserstoffperoxidumsatz von 99,6 % bestimmt.

Das Reaktionsgemisch enthält dann 6,0 g (48,3 mMol) 3-Methylbrenzkatechin und 2,95 g (23,8 mMol) 2-Methylhydrochinon, was einer Ausbeute an Dihydroxybenzolen von 72,4 %, bezogen auf umgesetztes Wasserstoffperoxid, entspricht.

**Patentansprüche**

1. Verfahren zur Herstellung von substituierten Dihydroxybenzolen sowie deren Monoäthern durch Kernhydroxylierung der entsprechenden Phenole bzw. Phenoläther mit Wasserstoffperoxid in organischem Lösungsmittel und in Gegenwart eines Katalysators, dadurch gekennzeichnet, daß man die Umsetzung bei Temperaturen von 20 - 200°C mit Lösungen von Wasserstoffperoxid, die wasserfrei sind, d.h. 0 - 1 Gew.-%, bevorzugt unter 0,5 Gew.-%, Wasser enthalten, die mit organischen Lösungsmitteln hergestellt werden, die mit Wasser Azeotrope bilden, deren Azeotropsiedepunkte unter dem Siedepunkt von Wasserstoffperoxid, bezogen auf Normaldruck, liegen, sowie in Gegenwart von Schwefeldioxid in Mengen von 0,0001 bis 0,1 Mol, bezogen auf 1 Mol Wasserstoffperoxid, durchführt, wobei das Molverhältnis von Phenol bzw. Phenoläther zu Wasserstoffperoxid bei 5 bis 20 : 1 liegt.

2. Verfahren nach Anspruch 1, dadurch gekennzeichnet, daß man Wasserstoffperoxidlösungen in Alkyl- oder Cycloalkylestern von gesättigten, aliphatischen Carbonsauren, die eine Gesamtkohlenstoffzahl von 4 - 8 besitzen, einsetzt.

3. Verfahren nach Anspruch 1 und 2, dadurch gekennzeichnet, daß man Wasserstoffperoxidlösungen von Estern der Essig- oder Propionsäure verwendet.

4. Verfahren nach Anspruch 1 bis 3, dadurch gekennzeichnet, dass man Wasserstoffperoxidlösungen in Essigsäure-n-propylester oder Essigsäure-i-propylester verwendet.

5. Verfahren nach Anspruch 1 bis 4, dadurch gekennzeichnet, dass man das Schwefeldioxid in Form einer Lösung der in Anspruch 2 genannten Alkyl- oder Cycloalkylester einsetzt.

6. Verfahren nach Anspruch 1, dadurch gekennzeichnet, dass man $SO_2$ in gasförmigem Zustand einsetzt.

7. Verfahren nach Anspruch 1 bis 6, dadurch gekennzeichnet, dass man Schwefeldioxid in Mengen von 0,0005 bis 0,01 Mol, bezogen auf 1 Mol Wasserstoffperoxid, einsetzt.

**Claims**

1. A process for the production of substituted dihydroxy benzenes and the mono ethers thereof by nuclear hydroxylation of the corresponding phenols or phenol ethers with hydrogen peroxide in an organic solvent and in the presence of a catalyst characterised in that the reaction is carried out at temperatures of from 20 to 200°C with solutions of hydrogen peroxide which are anhydrous, i.e. contain from 0 to 1 % by weight preferably less than 0.5 % of water which are produced with organic solvents and form with water azeotropes whose azeotropic boiling points lie below the boiling point of hydrogen peroxide with respect to normal pressure and in the presence of sulphur dioxide in quantities of from 0.0001 to 0.1 mol, with respect to 1 mol of hydrogen peroxide, the molar ratio of phenol or phenol ether to hydrogen peroxide being from 5 to 20:1.

2. A process according to claim 1 characterised in that hydrogen peroxide solutions in alkyl or cycloalkyl esters of saturated aliphatic carboxylic acids having a total carbon number of from 4 to 8 are used.

3. A process according to claims 1 and 2, characterised in that hydrogen peroxide solutions of esters of acetic or propionic acid are used.

4. A process according to claims 1 to 3, characterised in that hydrogen peroxide solutions in acetic acid-n-propyl ester or acetic acid-i-propyl ester are used.

5. A process according to claims 1 to 4, characterised in that the sulphur dioxide is used in the form of a solution of the alkyl or cycloalkyl ester mentioned in claim 2.

6. A process according to claim 1, characterised in that $SO_2$ is used in the gaseous state.

7. A process according to claims 1 to 6, characterised in that the sulphur dioxide is used in quantities of from 0.0005 to 0.01 mol with respect to 1 mol of hydrogen peroxide.

**Revendications**

1. Procédé pour la fabrication de dihydrobenzènes substitués, ainsi que de leurs monoéthers par hydroxylation sur le noyau des phénols, et phénoléthers, correspondants avec du peroxyde d'hydrogène dissous dans un solvant organique et en présence d'un catalyseur, procédé caractérisé en ce que la réaction est effectuée à des températures de 20 à 200°C, avec des solutions de peroxyde d'hydrogène qui sont exemptes d'eau, c'est-à-dire contenant 0 à 1 % de préférence moins de 0,5 % en poids d'eau, qui ont été préparées avec des solvants organiques qui forment avec l'eau des azéotropes dont le point d'ébullition azéotrope se situe en-dessous du point d'ébullition du peroxyde d'hydrogène, calculé a la pression normale, ainsi qu'en présence d'anhydride sulfureux dans des proportions de 0,0001 à 0,1 mole, calculé pour une mole de peroxyde d'hydrogène, le rapport moléculaire entre le phénol ou le phénoléther et le peroxyde d'hydrogène se situant de 5 à 20 : 1.

2. Procédé suivant la revendication 1, caractérisé en ce que l'on utilise des solutions de peroxyde d'hydrogène dans des esters alcoylés ou cycloalcoylés d'acides carboxyliques aliphatiques saturés qui possèdent un nombre total de carbones de 4 à 8.

3. Procédé suivant l'une des revendications 1 et 2, caractérisé en ce que l'on utilise des solutions de peroxyde d'hydrogène dans des esters des acides acétique ou propionique.

4. Procédé suivant les revendications 1 à 3, caractérisé en ce que l'on utilise des solutions de peroxyde d'hydrogène dans le n-propylester ou le i-propylester de l'acide acétique.

5. Procédé suivant les revendications 1 à 4, caractérisé en ce que l'on utilise l'anhydride sulfureux sous la forme d'une solution dans les esters alcoylés ou cycloalcoylés mentionnés dans la revendication 2.

6. Procédé suivant la revendication 1, caractérisé en ce que l'on utilise le $SO_2$ à l'état gazeux.

7. Procédé suivant l'une des revendications 1 à 6, caractérisé en ce que l'on utilise l'anhydride sulfureux dans une proportion de 0,0005 à 0,01 mol, calculé pour une mole de peroxyde d'hydrogène.